# EUROPEAN PATENT APPLICATION

(11) **EP 1 820 523 A1**
(43) Date of publication of application: **22.08.2007**
(21) Application number: 05806972.5
(22) Date of filing: 17.11.2005
(51) Int. Cl.: A61M 5/145, H01H 37/24, G05G 7/10

(54) **CONTROLLER UNIT**

(30) Priority: 18.11.2004 JP 2004334941
(71) Applicant: Nemoto Kyorindo Co., Ltd., Tokyo 113-0033 (JP)
(72) Inventor: NEMOTO, Shigeru c/o Nemoto Kyorindo Co., Ltd., Tokyo 1130033 (JP); HACHIYA, Takashi c/o Nemoto Kyorindo Co., Ltd., Tokyo 1130033 (JP)
(74) Representative: Cabinet Plasseraud
(86) International application number: PCT/JP2005/021131
(87) International publication number: WO 2006/054650

(57) **Abstract**

In a controller unit, controller cylinder 411 in which controller piston 412 and body cylinder 421 in which body piston 422 are connected to each other through tube member 413, and the slide position of body piston 422 is detected to control the operation of a movable mechanism. Since controller piston 412 is manually operated by sliding movement, delicate and accurate operation is enabled, an operating state can be easily maintained at a constant, and the durability of the controller unit can be improved.

## Description

### Technical Field

The present invention relates to a controller for controlling the operation of a movable mechanism mounted on an apparatus body of a movable apparatus, and more particularly, to a controller including a controller unit removably mounted on the apparatus body.

### Background Art

Presently available imaging diagnostic apparatuses for capturing diagnostic images of patients include CT (Computed Tomography) scanners, MRI (Magnetic Resonance Imaging) apparatuses, PET (Positron Emission Tomography) apparatuses, SPECT (Single Photon Emission Computed Tomography) apparatuses, ultrasonic diagnostic apparatuses and the like. Angiography apparatuses, MRA (MR angiography) apparatuses and the like are currently used as imaging diagnostic apparatuses for capturing vascular images of patients.

When the abovementioned imaging diagnostic apparatuses are used, a liquid such as a contrast medium or physiological saline may be injected into a patient. Chemical liquid injectors for automatically performing the injection have been put into practical use. A liquid syringe, for example including a syringe cylinder and a syringe piston slidably inserted into the syringe cylinder, is mounted on such a chemical liquid injector. A piston driving mechanism presses the syringe piston into the syringe cylinder.

The syringe cylinder is filled with a liquid and is connected to a blood vessel of a human body near the surface thereof through an extension tube and an injection needle. Thus, the liquid in the liquid syringe is injected with pressure into the blood vessel of the human body by the chemical liquid injector. Some of the chemical liquid injectors can automatically perform the injection in accordance with initial settings, and others can perform the injection based on real-time control. Some of the chemical liquid injectors performing the injection based on real-time control include a controller integral with the body of the injector, and others have a controller body of a controller formed separately from the body of the injector.

In such a case, for example, a manual operation member is slidably mounted on the controller body which contains a signal producing circuit connected to the manual operation member. The signal producing circuit comprises a variable resistor, for example, and produces a driving control signal in response to slide operation of the manual operation member.

The controller body is connected to one end of a flexible wire cable in which a conductor is covered, and the other end of the wire cable is connected to the body of the chemical liquid injector. The body of the injector includes a driving circuit for driving a slider mechanism. The driving circuit is connected to the signal producing circuit of the controller unit via the conductor of the wire cable.

In the chemical liquid injector described above, when an operator holds the controller body and slides the manual operation member, the signal producing circuit produces a driving control signal in response thereto and supplies the signal to the driving circuit in the body of the chemical liquid injector via the wire cable. The driving circuit controls the operation of the slider mechanism in response to the supplied driving control signal. Thus, the syringe piston of the liquid syringe is slid in accordance with the operation of the manual operation member of the controller.

In the abovementioned chemical liquid injector, the body of the chemical liquid injector and the controller body formed as separate components are connected to each other via the flexible wire cable, and the injection operation in the body of the chemical liquid injector can be manually operated by the controller body, so that excellent usability is achieved. In medical facilities where the abovementioned chemical liquid injector is used, an operator always needs to keep his hands and fingers clean because he manipulates the chemical liquid injector.

To this end, at least the controller body should be disinfected. However, it is difficult to disinfect the controller body containing the signal producing circuit and the like with disinfectant or fumigation. For example, if a controller unit removably mounted on the body of the chemical liquid injector is provided as a disposable component, the controller unit can always be kept clean.

However, the controller unit having the wire cable, the signal producing circuit and the like is not inexpensive, and in reality, it is difficult to form the controller unit as a disposable component. Therefore, as a controller to solve the problem, there is a product in which a spherical sealed container formed of an elastic member is mounted on a controller unit, and a hard sealed container and a pressure sensor are mounted on a body of a chemical liquid injector, and a flexible tube member connected to the sealed container on the controller unit is removably connected to the sealed container on the body of the injector.

In this controller, when an operator manually presses the sealed container on the controller unit, the pressure in the body of the injector is detected by the pressure sensor. The injection rate of the chemical liquid injector or the like can be controlled with the stress applied in the manual operation of the controller unit, for example (see patent document 1 below, for example).
Patent Document 1: Published Japanese Translation of PCT International Publication for Patent Application No. 2001-522659

### Disclosure of the Invention

### Subject to be Solved by the Invention

In the abovementioned controller, however, the stress applied to press the sealed container of the controller unit is used to control the operation of the movable mechanism, so that it is difficult to delicately and accurately operate the movable mechanism, and especially, it is extremely difficult to hold the operation state constant.

For example, when the injection rate of the chemical liquid injector is manually operated in accordance with the stress applied to press the sealed container of the controller unit, the injection rate is not easily maintained at a desired level. In addition, when the slide position of the syringe piston relative to the syringe cylinder of the liquid syringe is manually operated on the basis of the stress applied to press the sealed container of the controller unit, it is difficult to slide the syringe piston to a desired position and stop it there.

In the abovementioned controller, since the sealed container formed of the elastic member is manually pressed and deformed, the sealed container has a durability problem. The sealed container may be broken and inoperative during manual operation, by way of example.

The present invention has been made in view of the abovementioned problems, and it is an object thereof to provide a controller which includes a controller unit removably mounted on a body of a chemical liquid injector with ease of disinfection and the like in terms of structure, allows delicate and accurate operation, easily maintains a constant operation state, and provides excellent durability of the controller unit.

### Means to Solve the Subject

A controller according to the present invention includes a controller cylinder, a controller piston, a body cylinder, a body piston, a tube member, a position detecting means, and a driving control means to control the operation of a movable mechanism mounted on an apparatus body of a movable apparatus. The controller cylinder has a hollow structure which traps a fluid therein and is formed separately from the apparatus body. The controller piston is slidably inserted into the controller cylinder and manually operated. The body cylinder has a hollow structure which traps the fluid therein and is mounted on the apparatus body. The body piston is slidably inserted into the body cylinder. The tube member is flexible, connects the controller cylinder to the body cylinder, and flows the air therein. The position detecting means detects the slide position of the body piston. The driving control means controls the operation of the movable mechanism in accordance with the detected slide position. Thus, in the controller of the present invention, when the controller piston is manually slid relative to the controller cylinder, the body piston is slide relative to the body cylinder accordingly. The slide position is detected by the position detecting means, and the operation of the movable mechanism is controlled by the driving control means.

Various means referred to in the present invention may be arranged to perform their functions, and may comprise dedicated hardware for performing a predetermined function, a data processing apparatus whose predetermined function is given by a computer program, a predetermined function performed in a data processing apparatus according to a computer program, or a combination thereof.

Various means referred to in the present invention do not need to be a separate entity. A plurality of means may be constructed as one member, a certain means may be part of another means, or a certain means may have a portion overlapping a portion of another means.

### Effect of the Invention

In the controller of the present invention, when the controller piston is manually slid relative to the controller cylinder, the body piston is slide relative to the body cylinder accordingly. The slide position is detected by the position detecting means, and the operation of the movable mechanism is controlled by the driving control means, thereby controlling the operation of the movable mechanism in accordance with the slide position of the controller piston relative to the controller cylinder. As a result, the movable mechanism is delicately and accurately controlled with ease, and especially, the operation state is easily held constant. A sealed container made of an elastic member is not pressed or deformed by manual operation, so that the controller has excellent durability as a whole and the main portions are not broken to be inoperative during manual operation.

### Brief Description of the Drawings

Fig. 1 is a longitudinal section schematically showing the internal structure of a controller according to an embodiment of the present invention.
Fig. 2 is a longitudinal section schematically showing a controller piston pressed into a controller cylinder of the controller.
Fig. 3 is a longitudinal section showing a controller unit of the controller removed from an injection control unit corresponding to an apparatus body.
Fig. 4 is a perspective view showing the outer appearance of a chemical liquid injector.

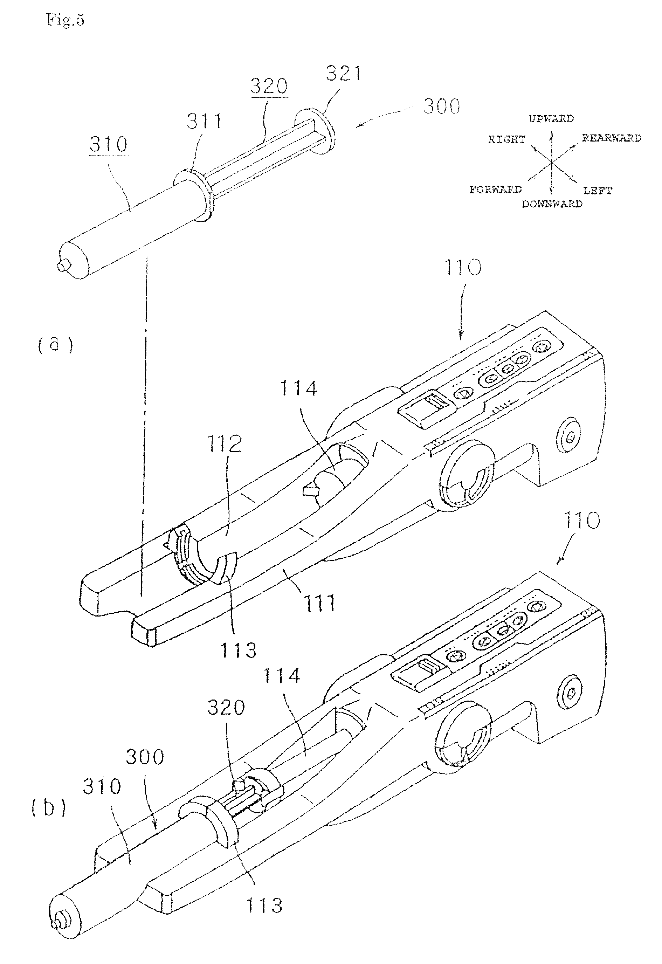

Fig. 5 is a perspective view showing how to mount a liquid syringe on an injection head of the chemical liquid injector.
Fig. 6 is a perspective view showing the outer appearance of an imaging diagnostic system.
Fig. 7 is a block diagram showing the circuit structure of the imaging diagnostic system.
Fig. 8 is a flow chart showing operation control of liquid injection in the chemical liquid injector.
Fig. 9 is a longitudinal section schematically showing the internal structure of a controller unit of a first modification.
Fig. 10 is a longitudinal section showing the internal structure of main portions of a controller of a second modification.

### Description of Reference Numerals

- 100: CHEMICAL LIQUID INJECTOR
- 101: INJECTION CONTROL UNIT corresponding to apparatus body
- 114: PISTON DRIVING MECHANISM serving as movable mechanism
- 130: COMPUTER BLOCK functioning as operation control means
- 300: LIQUID SYRINGE
- 310: SYRINGE CYLINDER
- 320: SYRINGE PISTON
- 400: CONTROLLER
- 410: CONTROLLER UNIT
- 411: CONTROLLER CYLINDER
- 412: CONTROLLER PISTON
- 413: TUBE MEMBER
- 421: BODY CYLINDER
- 422: BODY PISTON
- 423: LIGHT-EMITTING ELEMENT forming part of position detecting means
- 424: LIGHT-RECEIVING ELEMENT forming part of position detecting means
- 427: COIL SPRING serving as body biasing mechanism
- 520: COIL SPRING serving as controller biasing mechanism
- 540: PERMANENT MAGNET
- 550: HALL ELEMENT

### Best Mode for Carrying the Invention

### [Configuration of Embodiment]

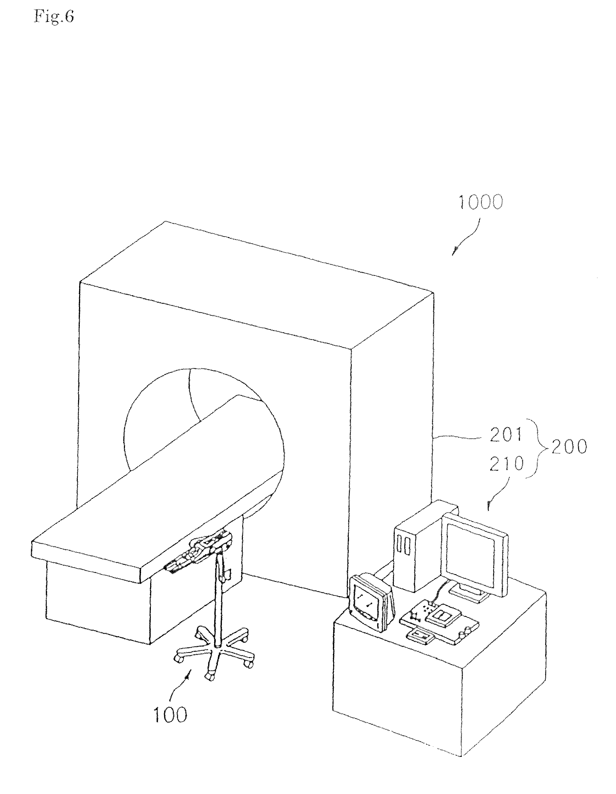

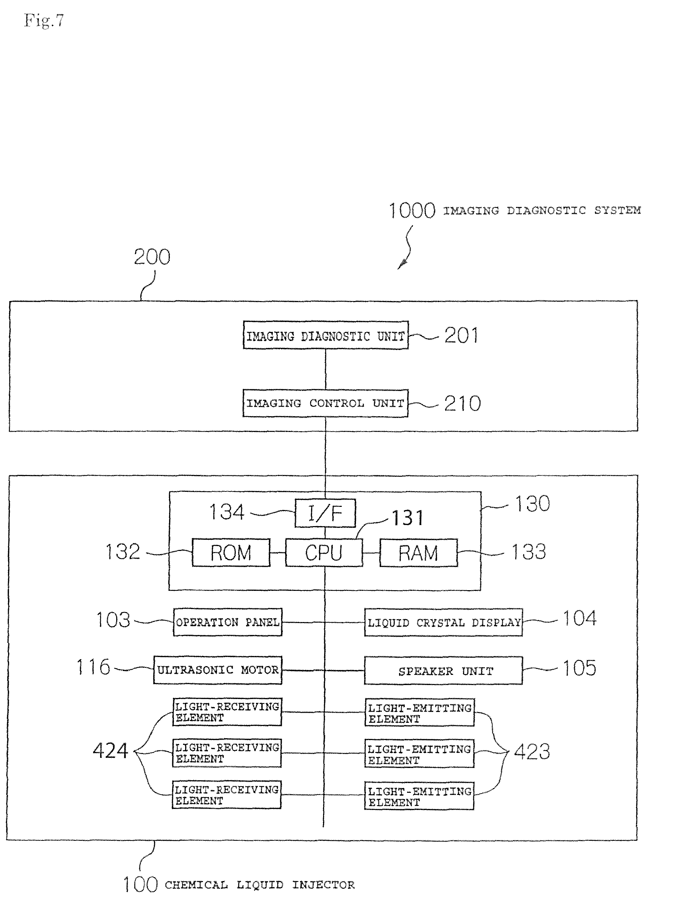

An embodiment of the present invention will hereinafter be described with reference to drawings. As shown in Figs. 6 and 7, imaging diagnostic system 1000 of the embodiment has chemical liquid injector 100 serving as a movable apparatus and MRI apparatus 200 serving as an imaging diagnostic apparatus. Chemical liquid injector 100 and MRI apparatus 200 are wire-connected.

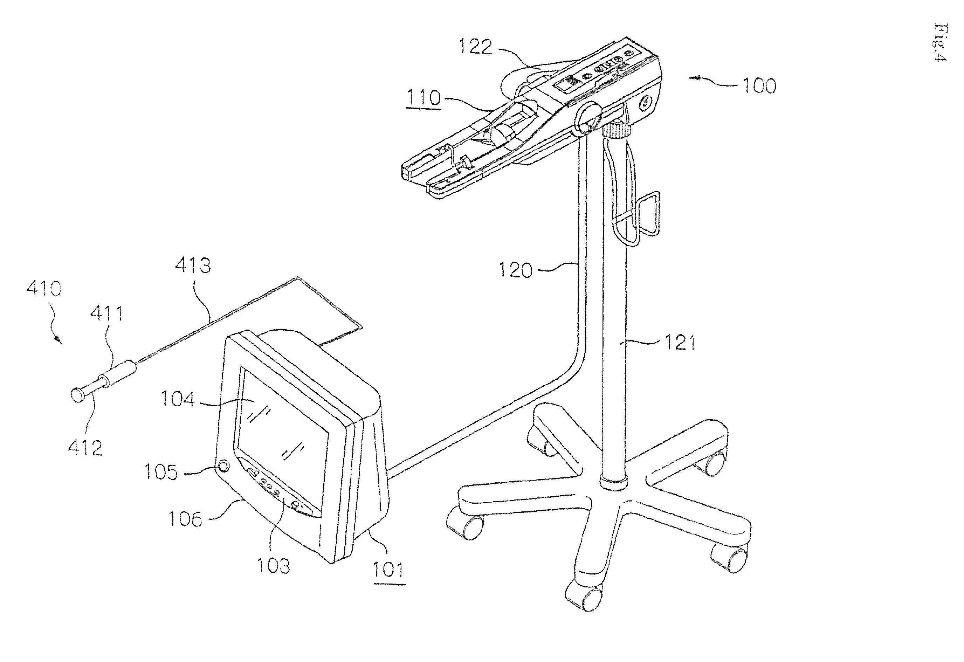

As shown in Fig. 4, injection control unit 101 and injection head 110 of chemical liquid injector 100, which correspond to an apparatus body, are constructed as separate components and are wire-connected to each other through communication cable 120. Injection head 110 is attached to the top end of caster stand 121 by movable arm 122. Head body 111 of injection head 110 has concave portion 112 in its upper surface.

Liquid syringe 300 includes syringe cylinder 310 and syringe piston 320 wherein syringe piston 320 is slidably inserted into syringe cylinder 310. Syringe cylinder 310 and syringe piston 320 have cylinder flange 311 and piston flange 321 formed in the outer circumferences of the trailing ends thereof, respectively. Syringe cylinder 310 has conduit 312 formed at its closed leading end.

In imaging diagnostic system 1000 of the embodiment, liquid syringe 300 is filled with a contrast medium as a liquid suitable for MRI apparatus 200. When chemical liquid injector 100 injects the contrast medium into a patient from liquid syringe 300, MRI apparatus 200 captures diagnostic images of the patient.

In chemical liquid injector 100 of the embodiment, syringe holding mechanism 113 which is openable or closable is formed in the forward section of concave portion 112 of injection head 110. Syringe holding mechanism 113 removably holds cylinder flange 311 of liquid syringe 300. Piston driving mechanism 114 serving as a movable mechanism is disposed in the rearward section of concave portion 112 of injection head 110. Piston driving mechanism 114 holds and slides syringe piston 320. Piston driving mechanism 114 has ultrasonic motor 116 as a driving source and slides syringe piston 320 with a screw mechanism (not shown) or the like.

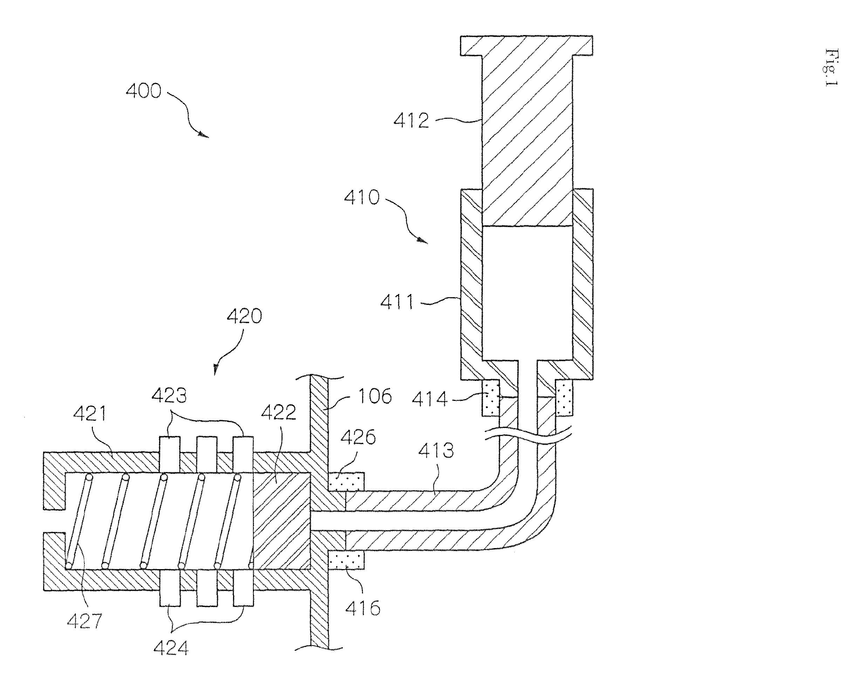

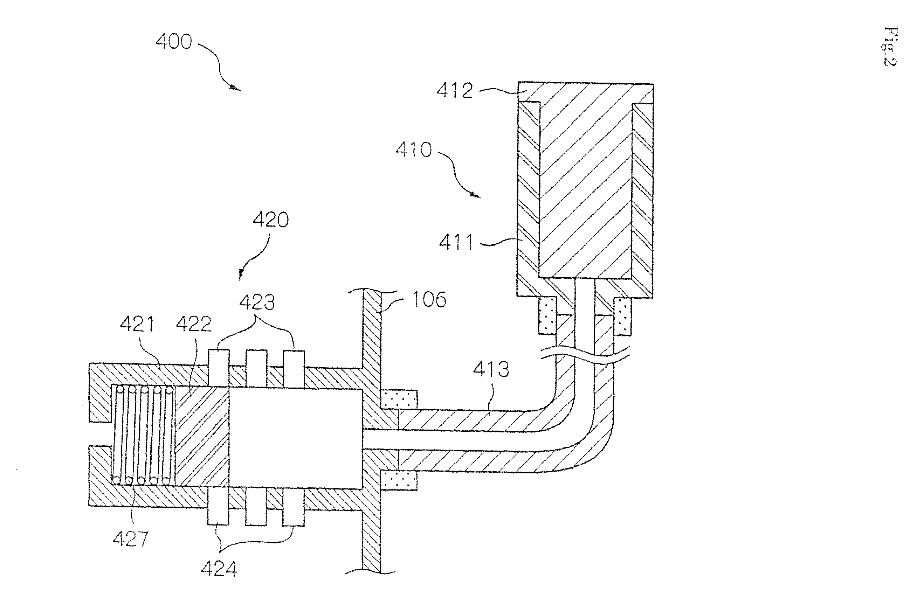

On the other hand, as shown in Fig. 4, injection control unit 101 has operation panel 103, liquid crystal display 104, speaker unit 105 and the like, all of which are disposed on the outer face of unit body 106. As shown in Figs. 1 and 2, controller unit 400 is integrally formed with injection control unit 101.

As shown in Fig. 7, injection control unit 101 contains computer block 130 functioning as a driving control means for controller 400. Computer block 130 is connected to components such as operation panel 103, liquid crystal display 104, speaker unit 105, ultrasonic motor 116, light-emitting elements 423, and light-receiving elements 424.

Computer block 130 is formed of a so-called one-chip microcomputer provided with hardware such as CPU (Central Processing Unit) 131, ROM (Read Only Memory) 132, RAM (Random Access Memory) 133, communication I/F (Interface) 134 and the like.

A computer program is installed in ROM 132. CPU 131 performs various types of processing in accordance with the computer program to integrate and control the components of chemical liquid injector 100. Although the components such as ultrasonic motor 116 and speaker unit 106 are actually connected to computer block 130 via a driving circuit and the like, Fig. 7 shows direct connections to simplify the description.

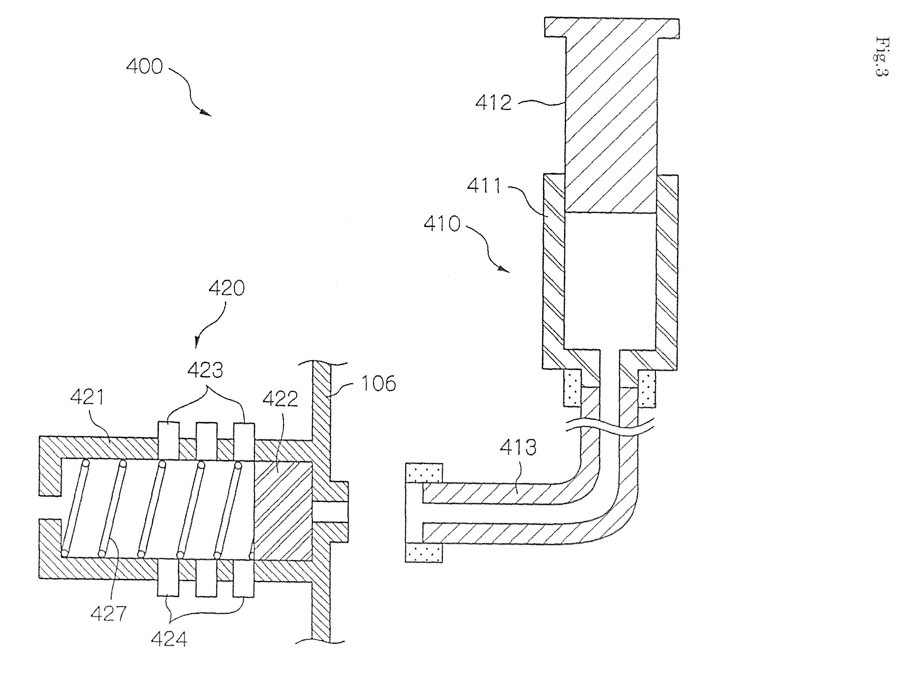

As shown in Figs. 1 to 3, controller 400 includes controller unit 410 and body unit 420. Body unit 420 is mounted integrally on injection control unit 101 of chemical liquid injector 100. On the other hand, controller unit 410 is formed separately from injection control unit 101, and is removably connected to controller unit 410 as shown in Fig. 3.

Controller unit 410 includes controller cylinder 411, controller piston 412, and tube member 413. Controller piston 412 is slidably inserted into controller cylinder 411 of a hollow structure which traps air as a fluid therein.

Controller cylinder 411 is formed in a cylindrical shape having a shape and dimensions suitable for holding by an operator, for example. Controller piston 412 is formed in a columnar shape which protrudes from controller cylinder 411 and has a shape and dimensions suitable for pressing by an operator's thumb. Tube member 413 is connected to controller cylinder 411 and flows air pressed thereinto from controller cylinder 411.

Body unit 420 includes body cylinder 421, body piston 422 and light-emitting/light-receiving elements 423, 424 serving as position detecting means. Body cylinder 421 is formed within unit body 106 of injection control unit 101 integrally therewith. More specifically, body cylinder 421 is formed in a hollow cylindrical shape which traps air as a fluid, similarly to controller cylinder 411. Body piston 422 is slidably inserted into body cylinder 421.

Body piston 422 is formed as a columnar shape sufficiently shorter and smaller than body cylinder 421 and is placed inside body cylinder 421 such that body piston 422 does not protrude therefrom. One end of body cylinder 421 is formed as conduit 426 which is opened to the outer surface of unit body 106. Tube member 413 of controller unit 410 is removably connected to conduit 426.

Connector members 414, 416 are put on both ends of tube member 413. Connector member 414 at the trailing end fixedly connects tube member 413 to controller cylinder 411, and connector member 416 at the leading end removably connects tube member 413 to conduit 426 of body cylinder 421.

In body cylinder 421 having conduit 426 formed at the one end, coil spring 427 serving as a body biasing mechanism is put at the other end. Coil spring 427 biases body piston 422 toward the one end from the other end of body cylinder 421.

A plurality of light-emitting elements 423 are arranged on the inner surface of body cylinder 421 such that they are sequentially shielded by body piston 422 as it slides. A plurality of light-receiving elements 424 are placed on the inner surface of body cylinder 421 such that they individually detect the light rays emitted by the plurality of light-emitting elements 423. Light-emitting and light-receiving elements 423, 424 are also connected to computer block 130 which controls the operation of ultrasonic motor 116 of piston driving mechanism 114 in response to the detection result of three light-receiving elements 424.

More particularly, in chemical liquid injector 100 of the embodiment, three light-emitting elements 423 and three light-receiving elements 424 are placed on body cylinder 421. Light-emitting and light-receiving elements 423, 424 are arranged at intervals such that one or two of them are sequentially shielded by body piston 422 as it slides.

In the initial state in which body piston 422 is in press contact with the one end of body cylinder 421 by the elastic force of coil spring 427, three light-receiving elements 424 individually detect the light rays from three light-emitting elements 423. When body piston 422 is pressed against the other end of body cylinder 421 by air to compress coil spring 427 to the limit, only the light ray from the third light-emitting element 423 is shielded. This is achieved by the arrangement of light-emitting and light-receiving elements 423, 424.

Computer block 130 forcedly stops piston driving mechanism 114 when all of the first to third light-receiving elements 424 detect light rays, and operates piston driving mechanism 114 at a first, lowest rate when the second and third light-receiving elements 424 detect light rays but the first light-receiving element 424 detects no light ray.

Computer block 130 operates piston driving mechanism 114 at a second rate when the first and second light-receiving elements 424 detect no light ray, at a third rate when only the second light-receiving element 424 detects no light ray, at a fourth rate when the second and third light-receiving elements 424 detect no light ray, and at a fifth, highest rate when only the third light-receiving element 424 detects no light ray.

The respective portions of controller unit 410 are made of material such as engineering plastics or the like which provides sufficient corrosion resistance, heat resistance, and strength, and which is not affected by magnetism.

### [Operation of the Embodiment]

The operation of imaging diagnostic system 1000 of the embodiment in the abovementioned structure will be described in order. First, as shown in Fig. 6, injection head 110 of chemical liquid injector 100 is disposed near imaging diagnostic unit 201 of MRI apparatus 200, and liquid syringe 300 filled with a liquid such as a contrast medium is prepared for use together with an extension tube (not shown) and the like.

Liquid syringe 300 is connected to a patient (not shown) in imaging diagnostic unit 201 via the extension tube or the like. Liquid syringe 300 is mounted on injection head 110 of chemical liquid injector 100. As shown in Fig. 3, controller piston 412 is pulled to the initial position from controller cylinder 411. Then, as shown in Figs. 1 and 4, tube member 413 of controller unit 410 is connected to body cylinder 421 of injection control unit 101.

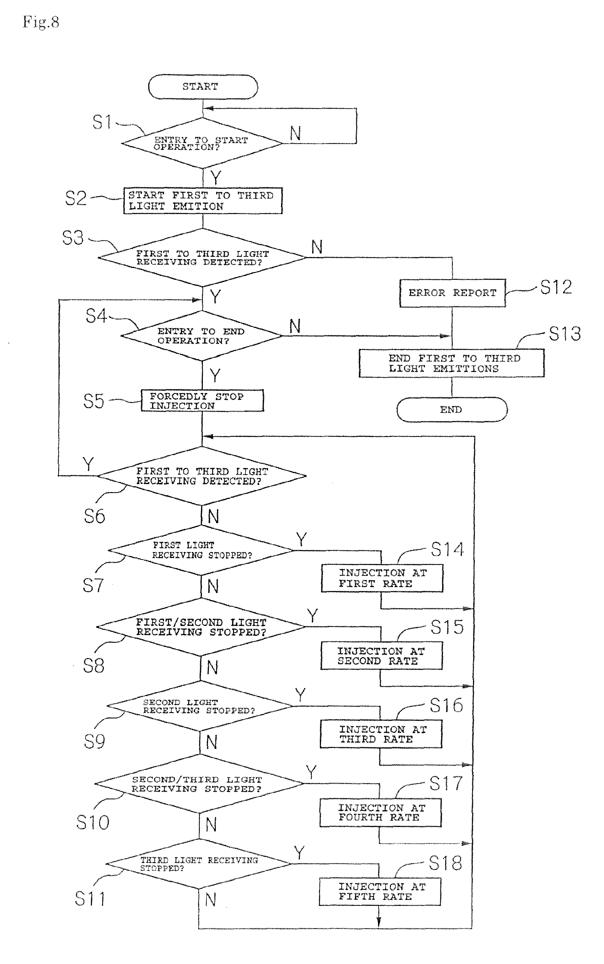

In this state, when an operator makes entry to start operation to chemical liquid injector 100 with operation panel 103 of injection control unit 101 or the like as shown in Fig. 8 (step S1), computer block 130 detects the entry and drives all of the first to third light-emitting elements 423 (step S2).

If light rays are not detected by all of the first to third light-receiving elements 424 (step S3), an error guidance such as "Abnormality occurring in controller. Check whether all of light-emitting elements emit light and whether body piston is placed at appropriate position" is output with display on liquid crystal display 104 and with sound from speaker unit 105 (step S12).

This can prevent start of injection operation when body piston 422 is not placed at the initial position or when any of light-emitting elements 423 fails to emit light. It should be noted that chemical liquid injector 100 of the embodiment can perform injection based on manual operation of operation panel 103 even when the abovementioned error state occurs.

On the other hand, when all of the first to third light-emitting elements 423 are driven and the light rays therefrom are detected by all of the first to third light-receiving elements 424 (steps S2, S3), computer block 130 holds piston driving mechanism 114 stopped as the initial state after the completion of the initial setting (step 5).

When the operator manually presses controller piston 412 into controller cylinder 411 of controller unit 410 in such a state, air is pressed into body cylinder 421 from controller cylinder 411 via tube member 413 to slide body piston 422 against the elastic force of coil spring 427.

Since one or two of the first to third light-receiving elements 422 are shielded to prevent the light reception depending on the slide position of body piston 422 (steps S7 to S11), computer block 130 drives piston driving mechanism 114 at the various rates described above in accordance with the detection result (steps S14 to 18).

More specifically, when controller piston 412 is pressed to a predetermined first stage to cause body piston 422 to shield only the first light-receiving element 424 (step S7), piston driving mechanism 114 is driven at the lowest first rate to inject the liquid into the patient at the lowest first rate (step S14).

Thereafter, when controller piston 412 is pressed to a second stage to cause body piston 422 to shield the first and second light-receiving elements 424 (step S8), piston driving mechanism 114 is driven at the second rate to inject the liquid into the patient at the second rate (step S15). In a similar manner, the injection rate of the liquid is increased every time controller piston 412 is pressed to a further stage manually by the operator.

Body piston 422 is biased to the initial position by coil spring 427, and the biasing acts on controller piston 412 in the form of air pressure. For this reason, controller piston 412 and body piston 422 are slid to return to the initial positions when the operator adjusts the pressure applied in the manual operation, thereby freely varying the injection speed of chemical liquid injector 100 with the manual operation of controller unit 410 by the operator.

When the operator completely releases the pressing in the manual operation, controller piston 412 and body piston 422 are returned to the initial positions. All of the first to third light-receiving elements 422 detect light rays, and piston driving mechanism 114 is forcedly stopped (steps S6, S5).

In this manner, the injection operation of chemical liquid injector 100 is forcedly stopped easily and immediately by manual operation of controller unit 410 by the operator. For example, if the operator inadvertently releases controller unit 410, the injection operation of chemical liquid injector 100 is forcedly stopped automatically.

### [Effect of the Embodiment]

In chemical liquid injector 100 of imaging diagnostic system 1000 of the embodiment, controller piston 412 of controller unit 410 can be manually operated to remotely control the liquid injection with piston driving mechanism 114. Controller unit 410 does not include light-emitting elements 423, light-receiving elements 424, or wires, and does not affect the magnetic field, so that chemical liquid injector 100 can be easily used near MRI apparatus 200.

In chemical liquid injector 100 of the embodiment, controller unit 410 which is actually operated manually is removably mounted on injection control unit 101 and does not include light-emitting elements 423, light-receiving elements 424, or wires. Thus, controller unit 410 removed from injection control unit 101 can be disinfected with a solution or boiling. Controller unit 410 which is actually operated manually can be kept clean at all times.

In controller 400 of the embodiment, since controller piston 412 is removably mounted on controller cylinder 411, the interiors of controller cylinder 411 and tube member 413 can be easily cleaned.

In addition, controller unit 410 is extremely inexpensive since it does not include light-emitting elements 423, light-receiving elements 424, or wires as described above. This makes it easy to form controller unit 410 as a disposable component which is discarded after it is used once or several times. Controller unit 410 can be kept clean more reliably.

In controller 400 of the embodiment, since tube member 413 is also removably mounted on controller cylinder 411, it is possible, for example, that controller cylinder 411 and controller piston 412 are cleaned and repeatedly used and tube member 413 is disposable and replaced after it is used once. Controller cylinder 411 and controller piston 412 are made of a hard material and have excellent durability, but tube member 413 is made of a flexible material and has less favorable durability, so that the abovementioned usage is advantageous.

In controller 400 of the embodiment, the pressure of trapped air is basically kept constant, and the air is moved to associate the slide position of controller piston 412 with the slide position of body piston 422 to realize the remote control.

Since the operation rate of piston driving mechanism 114 is controlled in stages by manually pressing slidable controller piston 412 into controller cylinder 411 in stages, piston driving mechanism 114 can be delicately and accurately operated with ease, and the operation state can be kept constant significantly easily.

A sealed container made of an elastic member is not pressed or deformed by manual operation as in the related art, so that the controller has excellent durability as a whole and the main portions are not broken to be inoperative during manual operation. In addition, parts of existing liquid syringe 200 can also be used as controller cylinder 411 and body cylinder 421, and controller piston 412 and body piston 422, which provides favorable productivity for controller 400.

In controller 400 of the embodiment, since body piston 422 shields one or two of three light-receiving elements 424 from receiving light depending on the slide position, the operation control is realized in the six stages from the stop state to the fifth rate based on the detection result with three light-receiving elements 424. Thus, the control accuracy is improved while the number of parts is reduced.

Coil spring 427 biases body piston 422 to the initial position, so that the injection rate of chemical liquid injector 100 can be freely increased or reduced by the operator increasing or reducing the pressure on controller piston 412. When the operator completely releases the pressing in manual operation, controller piston 412 and body piston 422 are returned to the initial positions, thereby making it possible to forcedly stop the injection operation of chemical liquid injector 100 easily and immediately. For example, if the operator inadvertently releases controller unit 410, the injection operation of chemical liquid injector 100 can be forcedly stopped automatically.

As shown in Fig. 3, when controller unit 410 is removed from body cylinder 421, body piston 422 is automatically returned to the initial position by the elastic force of coil spring 427. This eliminates the need to manually place body piston 422 to the initial position when controller unit 410 is put on body cylinder 421.

### [Modifications of the Embodiment]

The present invention is not in any way limited to the abovementioned embodiment, but various changes and modifications may be made therein without departing from the scope of the invention. For example, in the above embodiment, chemical liquid injector 100 is illustrated as the movable apparatus on which controller unit 410 is mounted to perform the operation control, but the present invention is applicable to various movable apparatuses.

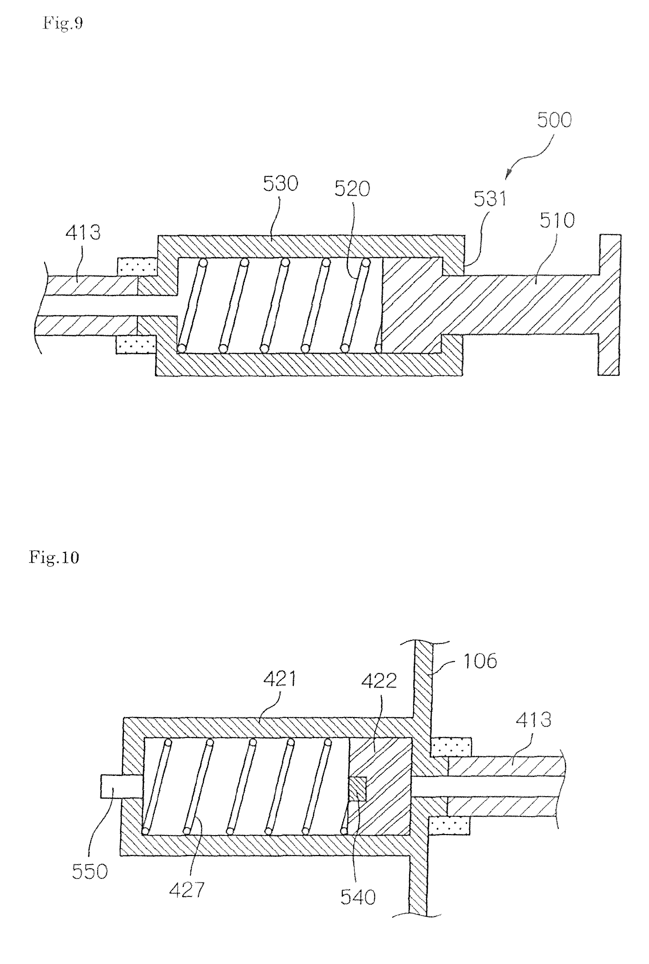

In the above embodiment, coil spring 427 inside body cylinder 421 is used to bias body piston 422 to the initial position. For example, as shown in Fig. 9, coil spring 520 serving as a controller biasing mechanism for biasing controller piston 510 of controller unit 500 to the initial position may be placed inside controller cylinder 530 to omit coil spring 427, or both of coil springs 427 and 520 may be used.

In this case, when controller unit 500 is removed from body cylinder 421, controller piston 510 is automatically returned to the initial position by the elastic force of coil spring 520, which eliminates the need to manually place controller piston 510 to the initial position when controller unit 500 is mounted on body cylinder 421.

In the above embodiment, the operation rate of piston driving mechanism 114 is associated with the slide position of controller piston 412 of controller unit 410. For example, the slide position of piston driving mechanism 114 on injection head 110 may be synchronized with the slide position of controller piston 412 of controller unit 410.

When the slide positions of controller piston 412 and piston driving mechanism 114 are synchronized in this manner, it is preferable that coil springs 427 and 520 for biasing body piston 422 and controller piston 412 to the initial positions, respectively, are omitted so that control piston 412 may be stopped at the position to which it is operated.

When controller piston 412 and body piston 422 are biased to the initial positions by coil springs 520 and 427 in the structure in which the slide positions of controller piston 412 and piston driving mechanism 114 are synchronized, it is preferable that the sliding of piston mechanism 114 is associated only with the sliding of controller piston 412 in the pressing direction and is not associated with the sliding of controller piston 412 in the returning direction.

In the above embodiment, controller piston 412 is removably inserted into controller cylinder 411. For example, as shown in Fig. 9, it is possible that stopper portion 531 is formed by protruding one end of controller cylinder 530 inward and is used to limit the moving range of controller piston 510.

In this case, it is difficult to freely remove controller piston 510 from controller cylinder 530, but controller piston 510 can be placed to the initial position easily and accurately, thereby preventing controller piston 510 from coming off controller cylinder 530.

In the above embodiment, light-emitting and light-receiving elements 423 and 424 are placed in the through-holes of body cylinder 421 to provide reliable optical connection between them. For example, it is possible that body cylinder 421 is formed of light-transmitting resin or glass and light-emitting and light-receiving elements 423 and 424 are placed outside body cylinder 421 (not shown).

In the above embodiment, a plurality of light-emitting and light-receiving elements 423 and 424 are arranged to detect the slide position of body piston 422. For example, as shown in Fig. 10, permanent magnet 540 may be put on body piston 422 and hall element 550 may be disposed on one end of body cylinder 421. In this case, since hall element 550 detects the distance from permanent magnet 540 to detect the slide position of body piston 422 in an analog fashion, so that the operation rate of piston driving mechanism 114 can be controlled steplessly.

In the above embodiment, only one liquid syringe 200 is mounted on injection head 110, and controller unit 410 is formed of one controller cylinder 411, one controller piston 412, and one tube member 413. It is possible that a plurality of liquid syringes 200 are mounted on the injection head and controller unit 410 includes the respective components arranged in parallel such that the number of the components is equal to the number of liquid syringes (not shown).

### Industrial Applicability

The present invention can be used as a controller for an apparatus including a movable mechanism.

## Claims

1. A controller for controlling operation of a movable mechanism mounted on an apparatus body of a movable apparatus, comprising:
a controller cylinder formed separately from the apparatus body and having a hollow structure which traps a fluid therein;
a controller piston slidably inserted into the controller cylinder and manually operated;
a body cylinder mounted on the apparatus body and having a hollow structure which traps the fluid therein;
a body piston slidably inserted into the body cylinder;
a flexible tube member connecting the controller cylinder to the body cylinder and flowing the air therein;
position detecting means for detecting a slide position of the body piston; and
driving control means for controlling the operation of the movable mechanism in accordance with the detected slide position.

2. The controller according to claim 1, wherein a controller unit including the controller cylinder, the controller piston and the tube member is formed separately from the apparatus body, and
the tube member of the controller unit is removably connected to the body cylinder in the apparatus body.

3. The controller according to claim 2, wherein the controller cylinder and the tube member are removably connected to each other.

4. The controller according to any one of claims 1 to 3, wherein the position detecting means includes:
a plurality of light-emitting elements which emit light rays to positions sequentially shielded by the body piston as the body piston slides; and
a plurality of light-receiving elements which detect the respective light rays emitted from the plurality of light-emitting elements.

5. The controller according to any one of claims 1 to 3, wherein the position detecting means includes:
a permanent magnet put on the body piston; and
a hall element placed at one end of the body cylinder in a slide direction of the body piston and detecting the distance from the permanent magnet.

6. The controller according to any one of claims 1 to 5, wherein the tube member is connected to one end of the body cylinder in a slide direction of the body piston,
further comprising a body biasing mechanism which biases the body piston toward the one end from the other end of the body cylinder.

7. The controller according to any one of claims 1 to 6, wherein the tube member is connected to one end of the controller cylinder in a slide direction of the controller piston,
further comprising a controller biasing mechanism which biases the controller piston toward the other end from the one end of the controller cylinder.

8. A controller unit for the controller according to claim 2, comprising:
a controller cylinder formed separately from the apparatus body and having a hollow structure which traps a fluid therein;
a controller piston slidably inserted into the controller cylinder and manually operated; and
a flexible tube member connecting the controller cylinder to the body cylinder and flowing the air therein.

9. A movable apparatus on which the controller unit according to claim 8 is removably mounted, comprising:
the apparatus body;
the movable mechanism mounted on the apparatus body;
a body cylinder mounted on the apparatus body and having a hollow structure which traps the fluid therein;
a body piston slidably inserted into the body cylinder;
position detecting means for detecting a slide position of the body piston; and
driving control means for controlling the operation of the movable mechanism in accordance with the detected slide position.

10. The movable apparatus according to claim 9, wherein the movable mechanism individually holds a syringe cylinder and a syringe piston of a liquid syringe, which includes the syringe cylinder and the syringe piston slidably inserted in the syringe cylinder, to move them relatively.

11. The movable apparatus according to claim 10, wherein the driving control means controls the operation of the movable mechanism such that an operation rate of the movable mechanism is synchronized with a slide position of the body piston detected by the position detecting means.

12. The movable apparatus according to claim 10, wherein the driving control means controls the operation of the movable mechanism such that a slide position of the syringe piston in the syringe cylinder is synchronized with a slide position of the body piston detected by the position detecting means.
